(19) Europäisches Patentamt
European Patent Office
Office européen des brevets

(11) Publication number: **0 095 294 A1**

(12) **EUROPEAN PATENT APPLICATION**

(21) Application number: 83302740.2

(22) Date of filing: 16.05.83

(51) Int. Cl.³: C 07 H 19/08
A 61 K 31/70

(30) Priority: 22.05.82 GB 8215019

(43) Date of publication of application: 30.11.83 Bulletin 83/48

(84) Designated Contracting States: BE CH DE FR GB IT LI NL SE

(71) Applicant: BEECHAM GROUP PLC
Beecham House Great West Road
Brentford Middlesex(GB)

(72) Inventor: Harnden, Michael Raymond
47 Guildford Road
Horsham Sussex(GB)

(72) Inventor: Luk, Kong
4 Palmer Close
Horley Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Deoxyuridine compounds, methods for preparing them and their use in medicine.**

(57) A compound of formula (I):

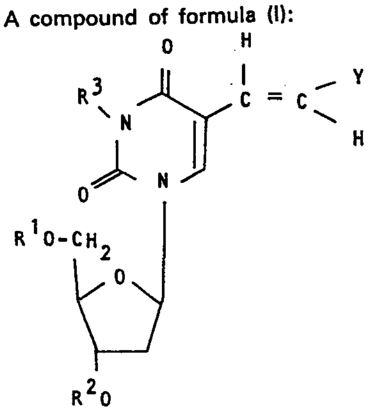

(I)

or a pharmaceutically acceptable salt or ester thereof, in which Y is a hydrogen or halogen atom, preferably a bromine atom, and each of $R^1$, $R^2$ and $R^3$ is a hydrogen atom; an acyl radical of the formula

X - C -,
O

in which X is a $C_{1-12}$ alkyl group an optionally substituted phenyl, or optionally substituted benzyl group, or a carboxy group of the formula

HO - C - Z -
O

in which Z is a branched or straight chain alkylene radical having from 1 to 4 carbon atoms in the chain; an optionally substituted $C_{1-12}$ alkoxycarbonyl group; an optionally substituted $C_{3-12}$ alkenyloxycarbonyl group; an optionally substituted phenoxycarbonyl group; or an optionally substituted phenyl $C_{1-6}$ alkoxycarbonyl group; provided at least one of $R^1$, $R^2$ and $R^3$ is an optionally substituted alkoxycarbonyl, optionally substituted alkenyloxycarbonyl, optionally substituted phenoxycarbonyl or optionally substituted phenyl $C_{1-6}$ alkoxycarbonyl group, is useful in treating virus infections.

EP 0 095 294 A1

0095294

# DEOXYURIDINE COMPOUNDS, METHODS FOR PREPARING THEM AND THEIR USE IN MEDICINE

This invention relates to certain deoxyuridine compounds which have antiviral activity.

Published European Patent Application No. 0 061 283 discloses esters of 5-(2-halogenovinyl)-2'-deoxyuridines which have antiviral activity selective against herpes viruses.

We have now found a group of mono-, di- and trisubstituted 5-(2-halogenovinyl)-2'-deoxyuridines which have excellent antiviral activity, and which are especially useful in the treatment of infections caused by herpes viruses, such as herpes simplex type 1, herpes simplex type 2 and varicella.

According to the present invention there is provided a compound of formula (I):

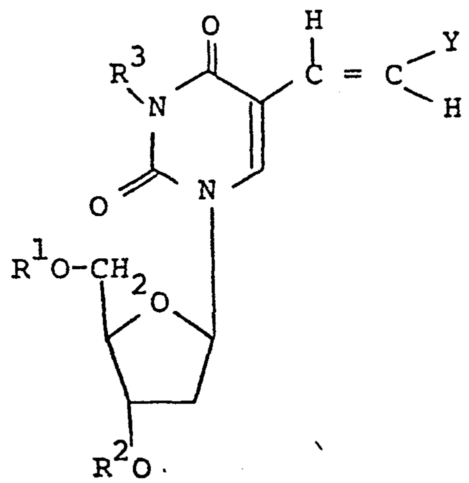

(I)

or a pharmaceutically acceptable salt or ester thereof,

in which Y is a hydrogen or halogen atom, and each of $R^1$, $R^2$ and $R^3$ is a hydrogen atom; an acyl radical of the formula $X - \underset{\underset{O}{\|}}{C} -$ , in which X is a $C_{1-12}$ alkyl group, an optionally substituted phenyl, or optionally substituted benzyl group, or a carboxy group of the formula $HO - \underset{\underset{O}{\|}}{C} - Z -$ in which Z is a branched or straight chain alkylene radical having from 1 to 4 carbon atoms in the chain; an optionally substituted $C_{1-12}$ alkoxycarbonyl group; an optionally substituted $C_{3-12}$ alkenyloxycarbonyl group; an optionally substituted phenoxycarbonyl group; or an optionally substituted phenyl $C_{1-6}$ alkoxycarbonyl group; provided at least one of $R^1$, $R^2$ and $R^3$ is an

optionally substituted alkoxycarbonyl, optionally substituted alkenyloxycarbonyl, optionally substituted phenoxycarbonyl or optionally substituted phenyl $C_{1-6}$ alkoxycarbonyl group.

Preferably, Y is a bromine atom

The $C_{1-12}$ alkoxy moiety may be branched or unbranched.

Preferred alkoxycarbonyl groups are $C_{1-6}$ alkoxy carbonyl groups, suitably methoxycarbonyl and ethoxycarbonyl.

When the alkoxy moiety contains two or more carbon atoms, the moiety may be optionally substituted by hydroxyl on any carbon atom not adjacent to the oxygen atom.

Preferred alkenyloxycarbonyl groups are $C_{3-6}$ alkenyloxycarbonyl groups such as propenyl.

Preferred phenoxycarbonyl groups are those wherein the optional substituents may be one or more stable moieties, such as halogen, nitro, alkoxy or alkyl.

Preferred phenyl $C_{1-6}$ alkoxycarbonyl groups are optionally substituted benzyloxycarbonyl groups, wherein the optional substituents may be one or more stable moieties as mentioned above.

Preferred acyl radicals are those wherein X is $C_{1-6}$ alkyl or Z is $(-CH_2-)_n$ wherein n is 2 or 3.

Preferred esters are those wherein one of $R^1$ or $R^2$ is a phosphate group and the other is optionally substituted alkoxycarbonyl, optionally substituted alkenyloxycarbonyl, optionally substituted phenoxycarbonyl or optionally substituted phenyl $C_{1-6}$ alkoxycarbonyl. Particularly preferred esters are those wherein $R^2$ is a phosphate group.

Preferred salts of the compounds of formula (I) are alkali metal salts of the $HO - \underset{\underset{O}{\|}}{C} - Z$ moiety, or alkali metal salts of the phosphate esters.

Particularly preferred compounds are those wherein $R^1$ or $R^2$ is an optionally substituted alkoxycarbonyl, optionally substituted alkenyloxycarbonyl, optionally substituted phenoxycarboxyl or optionally substituted phenyl $C_{1-6}$ alkoxycarboxy group.

Examples of compounds of the invention are as follows:

5-(E-2-Bromovinyl)-2'-deoxy-3',5'-bis-O-methoxycarbonyluridine;
5-(E-2-Bromovinyl)-2'-deoxy-3'-O-methoxycarbonyluridine;
5-(E-2-Bromovinyl)-2'-deoxy-5'-O-methoxycarbonyluridine;
5-(E-2-Bromovinyl)-2'-deoxy-3',5'-bis-O-ethoxycarbonyluridine;
5-(E-2-Bromovinyl)-2'-deoxy-5'-O-ethoxycarbonyluridine;
5-(E-2-Bromovinyl)-2'-deoxy-3'-O-ethoxycarbonyluridine;
5-(E-2-Bromovinyl)-2'-deoxy-3-ethoxycarbonyluridine;
5-(E-2-Bromovinyl)-2'-deoxy-5'-O-ethoxycarbonyl-3,3'-O-bisvaleryluridine;
5-(E-2-Bromovinyl)-2'-deoxy-5'-O-ethoxycarbonyl-3'-O-valeryluridine;

5-(E-2-Bromovinyl)-2'-deoxy-5'-O-ethoxycarbonyl-3-valeryluridine;

5-(E-2-Bromovinyl)-2'-deoxy-5'-O-ethoxycarbonyluridine 3'-Succinate Sodium Salt;

5-(E-2-Bromovinyl)-2'-deoxy-5'-O-ethoxycarbonyluridine 3'-Phosphate Disodium Salt;

5-(E-2-Bromovinyl)-2'-deoxy-5'-O-ethoxycarbonyluridine 3'-Phosphate Monosodium Salt;

5-(E-2-Bromovinyl)-2'-deoxy-5'-O-propoxycarbonyl-uridine;

5-(E-2-Bromovinyl)-5'-O-n-butoxycarbonyl-2'-deoxy-uridine;

5-(E-2-Bromovinyl)-3',5'-bis-O-butoxycarbonyl-2'-deoxyuridine;

5-(E-2-Bromovinyl)-5'-O-isobutoxycarbonyl-2'-deoxy-uridine;

5-(E-2-Bromovinyl)-5'-O-t-butoxycarbonyl-2'-deoxyuridine;

5-(E-2-Bromovinyl)-3',5'-bis-O-t-butoxycarbonyl-2'-deoxyuridine;

5-(E-2-Bromovinyl)-3'-O-t-butoxycarbonyl-2'-deoxyuridine;

5'-O-Allyloxycarbonyl-5-(E-2-bromovinyl)-2'-deoxyuridine;

5'-O-Benzyloxycarbonyl-5-(E-2-bromovinyl)-2'-deoxyuridine;

5-(E-2-Bromovinyl)-5'-O-phenoxycarbonyl-2'-deoxyuridine;

5-(E-2-Bromovinyl)-2'-deoxy-5'-O-(p-nitrophenoxy-carbonyl)uridine;

5-(E-2-Bromovinyl)-2'-deoxy-5'-O-isopropoxycarbonyl-uridine;

5-(E-2-Bromovinyl)-2'-deoxy-5'-O-(2-hydroxyethoxy-carbonyl)uridine;

2'-Deoxy-5'-O-ethoxycarbonyl-5-vinyluridine;

Compounds of formula (I) wherein $R^1$ or $R^2$ is optionally substituted $C_{1-12}$ alkoxycarbonyl, optionally substituted $C_{3-12}$ alkenyloxycarbonyl, optionally substituted phenoxycarbonyl or optionally substituted phenyl $C_{1-6}$ alkoxycarbonyl, and $R^3$ is a hydrogen atom, may be prepared by reacting a compound of formula (III):

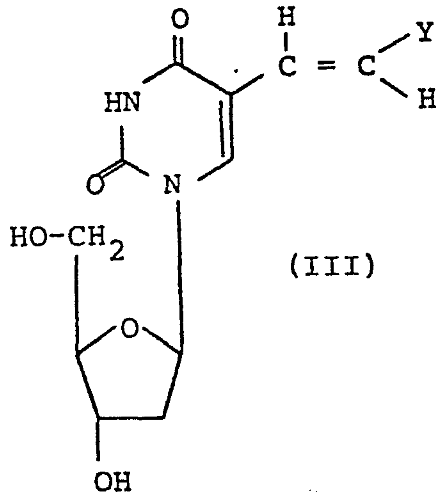

(III)

in which Y is as defined in formula (I), with the appropriate alkyl, alkenyl, phenyl or phenylalkyl chloroformate.

The reaction is suitably carried out in an anhydrous organic solvent in the presence of a base, preferably at depressed or room temperature, and the product purified chromato- graphically by, for example, column chromatography on silica gel.

Examples of suitable solvent systems for carrying out the reaction are anhydrous pyridine, tetrahydrofuran/sodium carbonate, and tetrahydrofuran/triethylamine.

The reaction will generally produce a mixture of products wherein both of $R^1$ and $R^2$ are alkoxy-, alkenyloxy-, phenoxy- or phenylalkoxy-carbonyl groups, and each of $R^1$ and $R^2$ is a hydrogen atom while the

other is alkoxy-, alkenyloxy-, phenoxy-or phenylalkoxy-carbonyl. The mixture can be separated into its pure components by chromatographic methods. The relative amounts of products will depend on the relative amounts of reactants, the physical conditions of the reaction, and the solvent system. For example, an excess of chloroformate will produce more of the disubstituted compound in the final mixture.

Compounds of formula (I) wherein $R^3$ is alkoxy-, alkenyloxy-, phenoxy- or phenylalkoxy-carbonyl as defined above, and $R^1$ and $R^2$ are each hydrogen atoms, may be prepared by treating a compound of formula (IV):

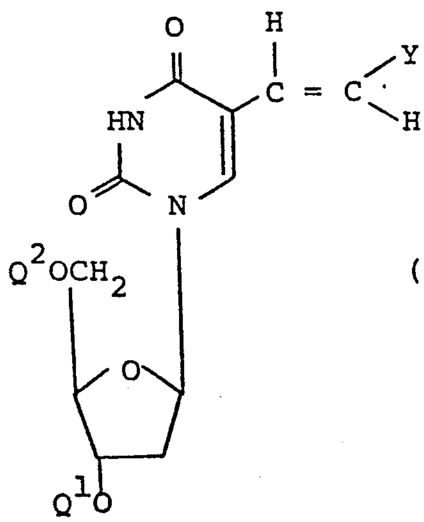

(IV)

wherein Y is as defined in formula (I), and $Q^1$ and $Q^2$ are O-protecting groups, with the appropriate alkyl-, alkenyl-, phenyl- or phenylalkyl-chloroformate, and deprotecting the resultant product.

Suitable O-protecting groups are trialkylsilyl groups, such as trimethylsilyl.

The reaction is suitably carried out in an anhydrous organic solvent in the presence of a base, preferably at room temperature, and the product may be purified by column chromatography on silica gel.

The compound of formula (IV) may be prepared by treating a compound of formula (III) as defined above, with a silylating agent.

Suitable silylating agents include halosilanes or silazanes of the formulae:

$L_3$ Si U; $L_3$ Si $NL_2$;
$L_3$ Si NH Si $L_3$; $L_3$ Si.NH.COL; $L_3$ Si.NH.CO.NH.Si $L_3$;
L NH.CO.NH.Si $L_3$; $L_3$ Si N = CLO Si $L_3$.

wherein U is a halogen and the various groups L which may be the same or different, each represents hydrogen or alkyl, aryl or aralkyl.

A preferred silylating agent is N,O-bistrimethylsilylacetamide, and the silylation reaction is preferably carried out in an anhydrous organic solvent, suitably tetrahydrofuran, at room temperature.

The product may be purified chromatographically by, for example, column chromatography on silica gel.

The protected compound of formula (IV), wherein $Q^1$ is replaced by a hydrogen atom, may also be used to prepare compounds of formula (I), wherein $R^2$ is alkoxy-, alkenyloxy-, phenoxy- or phenylalkoxy-carbonyl and $R^1$ and $R^3$ are each hydrogen atoms, by treatment with an alkyl-, alkenyl-, phenyl- or phenylalkyl-chloroformate and deprotecting the resultant product. The reaction conditions are similar to those described above.

Compounds of formula (I) wherein $R^1$, $R^2$ or $R^3$ is an acyl group as defined, may be prepared by treating the compounds of formula (I) wherein $R^1$, $R^2$ or $R^3$ is a hydrogen atom, with an acylating agent containing the 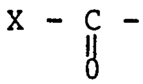 group or 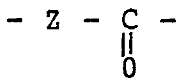 group, wherein X and Z are as defined with respect to formula (I).

A preferred acylating agent is an acid anhydride of formula 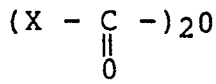 or 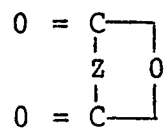

or an acid chloride of formula 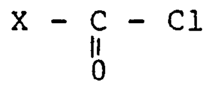

The reaction is conveniently carried out in an anhydrous organic solvent such as tetrahydrofuran in the presence of a base or pyridine, and the product purified by column chromatography on silica gel.

In the case where, in the reactant of formula (I), $R^1$ is alkoxy-, alkenyloxy-, phenoxy- or phenylalkoxy-carbonyl, and $R^2$ and $R^3$ are both hydrogen atoms, the reaction will generally produce a mixture of three products, wherein each one of the two hydrogen atoms is replaced by an acyl group while the other hydrogen atom remain intact, and wherein both hydrogen atoms are replaced by acyl groups.

The mixture can be separated into its pure components by chromatographic methods. In order to prepare compounds of formula (I) wherein two of $R^1$, $R^2$ and $R^3$ are different acyl groups, the mono-acylated compounds prepared as described above may be isolated from the three product mixture and further treated with a different acylating agent under similar reaction conditions. In this way, any combination of acylated derivatives may be prepared, as required.

Certain compounds of formula (I) can themselves be converted to other compounds of formula (I).

For example, compounds of formula (I) in which $R^1$ is optionally substituted phenoxycarbonyl may be converted into compounds wherein $R^1$ is $C_{1-12}$ alkoxycarbonyl by treatment with a $C_{1-12}$ alkanol, preferably in the presence of a basic organic solvent such as pyridine. In a similar manner, treatment with a $C_{2-12}$ diol, in place of the $C_{1-12}$ alkanol, will yield a compound of formula (I) wherein $R^1$ is hydroxy substituted $C_{2-12}$ alkoxycarbonyl.

Phosphate esters of the compounds of formula (I) may be prepared by treating a compound of formula (I), in which $R^1$ or $R^2$ is hydrogen, with phosphoryl chloride, preferably in an anhydrous basic organic solvent such as pyridine. The ester can be suitably prepared in the form of an alkali metal salt by the addition of an alkali metal bicarbonate to the ester.

The compounds of formula (I) or salts or esters thereof may be formulated for use in a pharmaceutical composition. Accordingly, in a further aspect of the invention, there is provided a pharmaceutical composition which comprises a compound of the formula (I) or a pharmaceutically acceptable salt or ester thereof, together with a pharmaceutically acceptable carrier or excipient.

Compositions which may be administered by the oral route to humans may be compounded in the form of syrups, tablets and capsules. When the composition is in the form of a tablet, any pharmaceutical carrier suitable for formulating such solid compositions may be used, for example magnesium stearate, starch, lactose,

glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the compounds of the invention may be made up into a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art.

Preferably, the compositions of this invention are in unit dosage form or in some other form that the patient may administer to himself a single dose. A suitable dosage unit might contain from 50 mg to 1 g of active ingredient, for example 100 to 500 mg. Such doses may be administered 1 to 4 times a day or more usually 2 or 3 times a day. The effective dose of compound depends on the particular compound employed, but is in general in the range of from 1.0 to 20 mg/kg of body weight per day or more usually 2.0 to 10 mg/kg per day.

In a further aspect of the invention there is provided a method of treating viral infections in human and non-human animals, which comprises administering to the sufferer an effective amount of a compound of formula (I) or a pharmaceutically acceptable salt or ester thereof.

The following Examples illustrate the invention.

5-(E-2-Bromovinyl)-2'-deoxy-3',5'-bis-O-methoxycarbonyluridine (Example 1), 5-(E-2-Bromovinyl)-2'-deoxy-3'-O-methoxycarbonyluridine (Example 2) and 5-(E-2-Bromovinyl)-2'-deoxy-5'-O-methoxycarbonyluridine (Example 3)

Methyl chloroformate (2ml) was added to a stirred mixture of 5-(E-2-bromovinyl)-2'-deoxyuridine (1.05g) and anhydrous sodium carbonate (1.5g) in dry tetrahydrofuran (30ml) at -30°. The mixture was allowed to come to room temperature and stirred at room temperature for 18 h. It was then partitioned between ethyl acetate and water. The organic extract was dried and evaporated and the residue was chromatographed over silica gel (40g). Elution with ethyl acetate-hexane (4:1) gave 5-(E-2-bromovinyl)-2'-deoxy-3',5'-bis-O-methoxycarbonyluridine (0.09g), m.p. 170-178° (from ethyl acetate-hexane), λmax (EtOH) 249 (ε 13,200) and 292 (ε 11,100) nm; νmax (KBr) 1760, 1745, 1690, and 1270 $cm^{-1}$; $\delta_H[(CD_3)_2SO]$ 2.50 (2H, m, 2'-$CH_2$), 3.74 (6H, s, $OCH_3$), 4.20 (1H, m, 4'-CH), 4.34 (2H, m, 5'-$CH_2$), 5.18 (1H, m, 3'-CH), 6.16 (1H, broad t, J 6Hz, 1'-CH), 6.85 (1H, d, J 14Hz, C<u>H</u>=CHBr), 7.29 (1H, d, J 14Hz, CH=C<u>H</u>Br), 7.84 (1H, s, 6-CH), and 11.64 (1H, broad s, $D_2O$ exchangeable, NH) (Found: C, 40.3; H, 3.50; N, 6.0 %. $C_{15}H_{17}N_2O_9Br$ requires C, 40.1; H, 3.8; N, 6.25 %); 5-(E-2-bromovinyl)-2'-deoxy-3'-O-methoxycarbonyluridine (0.17g), λmax (EtOH) 249 (ε 13,900) and 292 (ε 11,700) nm; νmax ($CHCl_3$) 1750, 1710, and 1280 $cm^{-1}$; $\delta_H$ ($CDCl_3$) 2.51 (2H, m, 2'-$CH_2$), 2.70 (1H, m, $D_2O$ exchangeable, OH), 3.84 (3H, s, $OCH_3$), 3.96 (2H, m, 5'-$CH_2$), 4.23 (1H, m, 4'-CH), 5.30 (1H, m, 3'-CH), 6.27 (1H, t, J 6Hz, 1'-CH), 6.66 (1H, d, J 14Hz, C<u>H</u>=CHBr), 7.35 (1H, d, J 14Hz, CH=C<u>H</u>Br), 7.87 (1H, s, 6-CH), and 9.22 (1H, broad s, $D_2O$ exchangeable, NH) (Found: C, 40.2; H, 3.75; N, 6.6 %. $C_{13}H_{15}N_2O_7Br$ requires C, 39.9; H, 3.85; N, 7.15 %); and 5-(E-2-bromovinyl)-2'-deoxy-5'-O-methoxycarbonyluridine (0.1g), m.p. 185-186° (from acetone-hexane), λmax (EtOH) 249 (ε 13,800) and 292 (ε 11,700) nm; νmax (KBr) 1758, 1742,

1710, 1690, 1678, 1390, 1365, and 1082 $cm^{-1}$; $\delta_H$ [$(CD_3)_2SO$] 2.22 (2H, t, J 6Hz, 2'-$CH_2$), 3.72 (3H, s, $OCH_3$), 3.83-4.40 (4H, m), 5.42 (1H, m, $D_2O$ exchangeable, OH), 6.16 (1H, t, J 6Hz, 1'-CH), 6.87 (1H, d, J 14Hz, C<u>H</u>=CHBr), 7.30 (1H, d, J 6Hz, CH=C<u>H</u>Br), 7.80 (1H, s, 6-CH), and 11.58 (1H, m, $D_2O$ exchangeable, NH) (Found: C, 40.1; H, 3.75; N, 7.1 %, M+ 390. $C_{13}H_{15}N_2O_7Br$ requires C, 39.9; H, 3.85; N, 7.15 % M+ 390).

Example 4

5-(E-2-Bromovinyl)-2'-deoxy-3',5'-bis-O-ethoxycarbonyl-uridine

Ethyl chloroformate (2.5ml) was added slowly to a stirred mixture of 5-(E-2-bromovinyl)-2'-deoxuridine (2.2g) and 4-dimethylaminopyridine (0.2g) in pyridine (30ml) at $0^{o}$. The mixture was then stirred at room temperature overnight. It was then poured into water and extracted with ethyl acetate. The organic extract was washed with aqueous hydrochloric acid, brine, aqueous sodium bicarbonate, dried, and evaporated. The residue was chromatographed over silica gel (80g). Elution of the column with ethyl acetate-hexane (1:1) gave the title compound (1.6g), m.p. 148-152$^{o}$ (from hexane-ethyl acetate), λmax (EtOH) 249 (ε 14,900) and 293 (ε 12,400) nm; νmax (KBr) 1750, 1712, 1682, and 1270 $cm^{-1}$; $\delta_H$ [$(CD_3)_2SO$] 1.24 (6H, t, J 7.2Hz, $-OCH_2C\underline{H}_3$), 2.45 (2H, m, 2'-$CH_2$), 4.15 (4H, q, J 7.2Hz, $-OC\underline{H}_2CH_3$), 4.33 (3H, m, 4'-CH, 5'-$CH_2$), 5.19 (1H, m, 3'-CH), 6.16 (1H, t, J 6.8Hz, 1'-CH), 6.84 (1H, d, J 13.7Hz, C$\underline{H}$=CHBr), 7.30 (1H, d, J 13.7Hz, CH=C$\underline{H}$Br), 7.84 (1H, s, 6-CH), and 11.62 (1H, m, $D_2O$ exchangeable, NH) (Found: C, 42.8; H, 4.3; N, 5.65 %. $C_{17}H_{21}N_2O_9Br$ requires C, 42.8; H, 4.45; N, 5.85 %).

Example 5

5-(E-2-Bromovinyl)-2'-deoxy-5'-O-ethoxycarbonyluridine

Ethyl chloroformate (1.5ml) was added slowly over 0.5 h to a stirred solution of 5-(E-2-bromovinyl)-2'-deoxyuridine (5g) in pyridine (100ml) at $0^{o}$ and the mixture was stirred at room temperature for 1 h. It was then partitioned between ethyl acetate and water. The organic extract was dried and evaporated, affording a colourless solid which was chromatographed over silica gel (150g). Elution with hexane-ethyl acetate (1:4) gave the title compound (2.5g), m.p. 198-$204^{o}$, λmax (EtOH) 293 (ε 12,400) and 249 (ε 14,700) nm; νmax (KBr) 1748, 1715, 1695, 1672, 1288, 1255, and 1078 $cm^{-1}$; $\delta_H$ [$(CD_3)_2SO$] 1.23 (3H, t, J 7.5Hz, $OCH_2C\underline{H}_3$), 2.20 (2H, broad t, J 6Hz, 2'-CH), 3.90-4.33 (6H, m), 5.45 (1H, $D_2O$ exchangeable, OH), 6.19 (1H, t, J 6Hz, 1'-CH), 6.90 (1H, d, J 14Hz, C$\underline{H}$=CHBr), 7.35 (1H, d, J 14Hz, CH=C$\underline{H}$Br), 7.83 (1H, s, 6-CH), and 11.7 (1H, broad s, NH) (Found: C, 41.6; H, 4.25; N, 6.8 %, M+ 404. $C_{14}H_{17}N_2O_7Br$ requires C, 41.5; H, 4.25; N, 6.9 %, M+ 404).

Example 6

5-(E-2-Bromovinyl)-2'-deoxy-3'-O-ethoxycarbonyluridine

Ethyl chloroformate (0.5ml) was added to a stirred solution of 5-(E-2-bromovinyl)-2'-deoxy-5'-O-(4,4'-dimethoxytrityl)uridine (1.4g) in pyridine (20ml) at $0^{o}$. The mixture was then stirred at room temperature for 1 h. It was then partitioned between ethyl acetate and water. The organic extract was dried and evaporated, affording an oil which was chromatographed over silica gel (60g). Elution with ethyl acetate-hexane (1:1) gave 5-(E-2-bromovinyl)-2'-deoxy-5'-O-(4,4'-dimethoxytrityl)-3'-O-ethoxycarbonyluridine (0.85g), λmax (EtOH) 235 (ε 29,400) and 283 (ε 11,700) nm; νmax ($CHCl_3$) 1720, 1690, and 1255 $cm^{-1}$; $\delta_H$ ($CDCl_3$) 1.30 (3H, t, J 7.5Hz, $OCH_2C\underline{H}_3$), 2.23-2.80 (2H, m, 2'-$CH_2$), 3.48 (2H, m, 5'-$CH_2$), 4.22 (2H, q, J 7.5Hz, $OC\underline{H}_2CH_3$), 4.26 (1H, m, 4'-CH), 5.38 (1H, m, 3'-CH), 5.81 (1H, d, J 14Hz, C$\underline{H}$=CHBr), 6.43 (1H, m, 1'-CH), 6.89 (4H, d, J 9Hz, ArH), 7.20-7.44 (10H, m, ArH, CH=C$\underline{H}$Br), 7.73 (1H, s, 6-CH), and 8.86 (1H, broad s, $D_2O$ exchangeable, NH) (Found: C, 59.1; H, 4.75; N, 3.9 %. $C_{35}H_{35}N_2O_9Br$ requires C, 59.40; H, 5.0; N, 3.95 %).

A solution of the 5-(E-2-bromovinyl)-2'-deoxy-5'-O-(4,4'-dimethoxytrityl)-3'-O-ethoxycarbonyluridine in 80% aqueous acetic acid (20ml) was stirred at room temperature for 0.75 h. The mixture was then partitioned between ethyl acetate and water. The organic extract was washed with water, bicarbonate, brine, dried and evaporated. The residue was chromatographed over silica gel (30g). Elution of the column with ethyl acetate-hexane (3:2) gave the title compound (0.36g), λmax 249 (ε 14,000) and 292 (ε 11,800) nm; νmax ($CHCl_3$) 1710 (broad) and 1270 $cm^{-1}$; $\delta_H$ ($CDCl_3$) 1.32 (3H, t, J 7.5Hz, $OCH_2C\underline{H}_3$), 2.47 (2H, m, 2'-$CH_2$), 2.82 (1H, m, $D_2O$ exchangeable, OH), 3.97 (2H, m, 5'-$CH_2$), 4.22 (2H, q, J 7.5Hz, $OC\underline{H}_2CH_3$), 4.25 (1H, m, 4'-CH), 5.29 (1H, m, 3'-CH), 6.29 (1H, broad t, J 6Hz, 1'-CH), 6.66 (1H, d, J 14Hz, C$\underline{H}$=CHBr), 7.36 (1H, d, J 14Hz, CH=C$\underline{H}$Br), 7.91 (1H, s, 6-CH), and 9.45 (1H, m, $D_2O$ exchangeable, NH)

(Found: C, 41.85; H, 4.0; N, 6.5 %. $C_{14}H_{17}N_2O_7Br$ requires C, 41.5; H, 4.25; N, 6.9 %).

Example 7

5-(E-2-Bromovinyl)-2'-deoxy-3-ethoxycarbonyluridine

To a stirred solution of 3',5'-bis-O-trimethylsilyl-5-(E-2-bromovinyl)-2'-deoxyuridine (0.6g) in tetrahydrofuran (15ml), 4-dimethylaminopyridine (0.01g) and triethylamine (0.5ml) were added followed by ethyl chloroformate (0.2ml). The mixture was stirred at room temperature for 2 h. Further ethyl chloroformate (0.2ml) was then added and the mixture stirred at room temperature for another 2 h. It was then partitioned between ethyl acetate and water. The organic extract was dried and evaporated, affording an oil which was chromatographed over silica gel (20g). Elution of the column with n-hexane-ethyl acetate (1:4) gave the title compound, m.p. 136-140$^{o}$ (ethyl acetate-hexane), λmax (EtOH) 250 (ε 14,300) and 297 (ε 11,200) nm; νmax (KBr) 1794, 1732, 1660, 1460, 1282, and 1232 $cm^{-1}$; $\delta_H$ [$(CD_3)_2SO$] 1.30 (3H, t, J 7.5Hz, $OCH_2C\underline{H}_3$), 2.22 (2H, m, 2'-$CH_2$), 3.62 (2H, m, 5'-$CH_2$), 3.83 (1H, m, 4'-CH), 4.25 (1H, m, 3'-CH), 4.42 (1H, q, J 7.5Hz, $OC\underline{H}_2CH_3$), 5.03-5.20 (2H, m, OH), 6.10 (1H, t, J 6Hz, 1'-CH), 6.87 (1H, s, J 14Hz, C$\underline{H}$=CHBr), 7.23 (1H, d, J 14Hz, CH=C$\underline{H}$Br), and 8.23 (1H, s, 6-CH) (Found: C, 41.0; H, 4.35; N, 6.85 %, M+ 404. $C_{14}H_{17}N_2O_7Br$ requires C, 41.5; H, 4.25; N, 6.9 %, M+ 404).

Examples 8, 9 and 10

0095294

5-(E-2-Bromovinyl)-2'-deoxy-5'-O-ethoxycarbonyl-3,3'-O-bisvaleryluridine (Example 8), 5-(E-2-Bromovinyl)-2'-deoxy-5'-O-ethoxycarbonyl-3'-O-valeryluridine (Example 9) and 5-(E-2-Bromovinyl)-2'-deoxy-5'-O-ethoxycarbonyl-3-valeryluridine (Example 10)

To a stirred solution of 5-(*E*-2-bromovinyl)-2'-deoxy-5'-O-ethoxycarbonyluridine (0.46g) in tetrahydrofuran (20ml) at $0^{o}$, triethylamine (0.24ml) was added followed by valeryl chloride (0.15ml) and the mixture was stirred at $0^{o}$ for 0.5 h. A further portion of triethylamine (0.24ml) and valeryl chloride (0.15ml) was added and the mixture stirred for another 0.5 h at $0^{o}$. It was then partitioned between ethyl acetate and water. The organic extract was dried and evaporated, affording an oil which was chromatographed over silica gel (20g). Elution with n-hexane-ethyl acetate gave 5-(*E*-2-bromovinyl)-2'-deoxy-5'-O-ethoxycarbonyl-3,3'-O-bisvaleryluridine (0.12g), νmax ($CHCl_3$) 1790, 1750, 1730, 1710, 1670, and 1260 $cm^{-1}$; $\delta_H$ ($CDCl_3$) 0.93 (6H, t, J 7Hz), 1.40 (3H, t, J 7.5Hz, $OCH_2C\underline{H}_3$), 1.14-1.90 (8H, m), 2.10-2.62 (4H, m), 2.60 (2H, t, J 7.5Hz), 4.15-4.47 (5H, m), 5.24 (1H, m, 3'-CH), 6.37 (1H, m, 1'-CH), 6.69 (1H, d, J 14Hz, C$\underline{H}$=CHBr), 7.38 (1H, d, J 14Hz, CH=C$\underline{H}$Br), and 7.73 (1H, s, 6-CH); and 5-(*E*-2-bromovinyl)-2'-deoxy-5'-O-ethoxycarbonyl-3'-O-valeryluridine (0.05g), νmax ($CHCl_3$) 1750, 1710, and 1260 $cm^{-1}$; $\delta_H$ ($CDCl_3$) 0.90 (3H, t, J 7Hz), 1.35 (3H, t, J 7.5Hz, $OCH_2C\underline{H}_3$), 1.14-1.74 (4H, m), 2.02-2.60 (4H, m), 4.13-4.60 (5H, m), 5.30 (1H, m, 3'-CH), 6.40 (1H, m, 1'-CH), 6.67 (1H, d, J 14Hz, C$\underline{H}$=CHBr), 7.40 (1H, d, J 14Hz, CH=C$\underline{H}$Br), 7.70 (1H, s, 6-CH), and 9.57 (1H, m, $D_2O$ exchangeable, NH); followed by 5-(*E*-2-bromovinyl)-2'-deoxy-5'-O-ethoxycarbonyl-3-valeryluridine (0.25g), νmax ($CHCl_3$) 1790, 1750, 1710, 1360, and 1290 $cm^{-1}$; $\delta_H$ ($CDCl_3$) 0.89 (3H, t, J 7Hz), 1.34 (3H, t, J 7.5Hz, $OCH_2C\underline{H}_3$), 1.10-1.84 (4H, m), 1.97-2.55 (2H, m, 2'-$CH_2$), 2.77 (2H, t, J 7.5Hz), 3.34 (1H, m, $D_2O$ exchangeable, OH), 4.05-4.50 (6H, m), 6.28 (1H, t, J 6Hz, 1'-CH), 6.70 (1H, d, J 14Hz, C$\underline{H}$=CHBr), 7.36 (1H, d, J 14Hz, CH=C$\underline{H}$Br), and 7.76 (1H, s, 6-CH).

Example 11

## 5-(E-2-Bromovinyl)-2'-deoxy-5'-O-ethoxycarbonyluridine 3'-Succinate Sodium Salt

A mixture of 5-(*E*-2-bromovinyl)-2'-deoxy-5'-O-ethoxycarbonyluridine (0.11g), 4-dimethylaminopyridine (0.1g), and succinic anhydride (0.05g) in pyridine (2ml) was stirred at room temperature for 70 h. It was then evaporated to dryness. The residue was partitioned between aqueous hydrochloric acid and ethyl acetate. The organic layer was washed with water and then extracted with water at pH 7.5. The aqueous extract was concentrated to 4ml and filtered through 2 $C_{18}$ Sep-Pak cartridges, elution with aqueous methanol gave the title compound (0.09g), $\lambda$max ($H_2O$) 249 ($\varepsilon$ 14,400) and 293 ($\varepsilon$ 10,900) nm; $\nu$max (KBr) 1740, 1710, 1690, 1585, 1285, and 1260 $cm^{-1}$; $\delta_H$ ($D_2O$) 1.20 (3H, t, J 7.5Hz, 2'-$CH_2$), 2.10-2.73 (6H, m), 4.00-4.43 (5H, m), 5.23 (1H, m, 3'-CH), 6.20 (1H, m, 1'-CH), 6.54 (1H, d, J 14Hz, C*H*=CHBr), 7.10 (1H, d, J 14Hz, CH=C*H*Br), and 7.60 (1H, s, 6-CH).

Example 12

5-(E-2-Bromovinyl)-2'-deoxy-5'-O-ethoxycarbonyluridine 3'-Phosphate Disodium Salt

Phosphoryl chloride (0.03ml) in pyridine (1ml) was added to a stirred solution of 5-(E-2-bromovinyl)-2'-deoxy-5'-O-ethoxycarbonyluridine (0.11g) in pyridine (2ml) at room temperature and the mixture was stirred at room temperature for 0.5 h. It was evaporated to dryness and the residue dissolved in water (10ml). Sodium bicarbonate (0.125g) was then added and the solution was evaporated to dryness. The residue was filtered through 2 $C_{18}$ Sep-Pak cartridges. Elution with aqueous methanol gave the title compound (0.05g), νmax (KBr) 1745, 1710, 1690, 1285, 1260, 1085, 1010, and 945 $cm^{-1}$; $\delta_H$ [$(CD_3)_2SO$] 1.22 (3H, t, J 7.5Hz, $OCH_2C\underline{H}_3$), 2.38 (2H, m, 2'-$CH_2$), 3.96-4.55 (5H, m, 5'-$CH_2$, 4'-CH, $OC\underline{H}_2CH_3$), 4.83 (1H, m, 3'-CH), 6.20 (1H, m, 1'-CH), 6.85 (1H, d, J 14Hz, C$\underline{H}$=CHBr), 7.31 (2H, d, J 14Hz, CH=C$\underline{H}$Br), 7.88 (1H, s, 6-CH), and 11.60 (1H, m, $D_2O$ exchangeable, NH).

Example 13

5-(E-2-Bromovinyl)-2'-deoxy-5'-O-ethoxycarbonyluridine 3'-Phosphate Monosodium Salt

Phosphoryl chloride (0.03ml) in pyridine (1ml) was added to a stirred solution of 5-(*E*-2-bromovinyl)-2'-deoxy-5'-O-ethoxycarbonyluridine (0.11g) in pyridine (2ml) at room temperature and the mixture was stirred at room temperature for 0.5 h. It was evaporated to dryness and the residue dissolved in water (10ml). Sodium bicarbonate (0.1g) was then added and the solution was evaporated to dryness. The residue was filtered through 2 $C_{18}$ Sep-Pak cartridges. Elution with aqueous methanol gave the title compound (0.04g), $\delta_H$ ($D_2O$) 1.20 (3H, t, J 7.5Hz, $OCH_2C\underline{H}_3$), 2.40 (2H, m, 2'-$CH_2$), 4.00-4.53 (5H, m, 5'-$CH_2$, 4'-CH, $OC\underline{H}_2CH_3$), 4.73 (1H, m, 3'-CH), 6.20 (1H, t, J 6.5Hz, 1'-CH), 6.63 (1H, d, J 14Hz, C$\underline{H}$=CHBr), 7.08 (1H, d, J 14Hz, CH=C$\underline{H}$Br), and 7.65 (1H, s, 6-CH).

Example 14

5-(E-2-Bromovinyl)-2'-deoxy-5'-O-propoxycarbonyluridine

Propyl chloroformate (0.5ml) was added to a solution of 5-(E-2-bromovinyl)-2'-deoxyuridine (1.22g) in pyridine (20ml) at $0^{o}$ and the mixture was stirred at $0^{o}$ for 1 h. It was then partitioned between water and ethyl acetate. The organic extract was washed with aqueous hydrochloric acid, brine, sodium bicarbonate, dried, and evaporated. The residue was chromatographed over silica gel (60g). Elution of the column with ethyl acetate-hexane (9:1) gave the title compound (1.0g), m.p. 171-173$^{o}$ (from ethyl acetate-ether), λmax (EtOH) 249 (ε 14,400) and 293 (ε 12,400) nm; νmax (KBr) 1750, 1710, 1690, 1675, 1470, and 1282 $cm^{-1}$; $\delta_H$ [$(CD_3)_2SO$] 0.90 (3H, t, J 7.5Hz), 1.63 (2H, m), 2.30 (2H, t, J 6Hz, 2'-$CH_2$), 3.83-4.40 (6H, m), 5.40 (1H, m, $D_2O$ exchangeable, CH), 6.34 (1H, t, J 6Hz, 1'-CH), 6.83 (1H, d, J 14Hz, CH=CHBr), 7.28 (1H, d, J 14Hz, CH=CHBr), 7.77 (1H, s, 6-CH), and 11.55 (1H, m, $D_2O$ exchangeable, NH) (Found: C, 43.2; H, 4.45; N, 6.5 %. $C_{15}H_{19}N_2O_7Br$ requires C, 42.95; H, 4.55; N, 6.7 %).

Example 15

5-(E-2-Bromovinyl)-5'-O-n-butoxycarbonyl-2'-deoxyuridine

n-Butyl chloroformate (0.7g) was added to a solution of 5-(E-2-bromovinyl)-2'-deoxyuridine (1.43g) in pyridine (25ml) at $0^{o}$ and the mixture was stirred at room temperature for 1 h. The same work-up and chromatography as in Example 14 gave the title compound (1.2g), m.p. 150-$153^{o}$ (from ethyl acetate-hexane), λmax (EtOH) 249 (ε 12,900) and 293 (ε 11,200) nm; νmax (KBr) 1750, 1715, 1685, 1275, and 1160 $cm^{-1}$; $\delta_H$ [$(CD_3)_2SO$] 0.87 (3H, t, J 7.5Hz), 1.10-1.76 (4H, m), 2.17 (2H, t, J 6.5Hz, 2'-$CH_2$), 3.80-4.40 (6H, m), 5.40 (1H, m, $D_2O$ exchangeable, OH), 6.33 (1H, t, J 6.5Hz, 1'-CH), 6.82 (1H, d, J 14Hz, CH=CHBr), 7.27 (1H, d, J 14Hz, CH=CHBr), 7.75 (1H, s, 6-CH), and 11.56 (1H, m, $D_2O$ exchangeable, NH) (Found: C, 44.8; H, 5.0; N, 6.4 %. $C_{16}H_{21}N_2O_7Br$ requires C, 44.35; H, 4.9; N, 6.45%).

Examples 16 and 17

5-(E-2-Bromovinyl)-3',5'-bis-O-isobutoxycarbonyl-2'-deoxyuridine (Example 16) and 5-(E-2-Bromovinyl)-5'-O-isobutoxycarbonyl-2'-deoxyuridine (Example 17)

isoButyl chloroformate (1.6ml) was added to a stirred solution of 5-(E-2-bromovinyl)-2'-deoxyuridine (2.05g) in pyridine (30ml) at $0^{o}$ and the mixture was stirred at room temperature for 1 h. It was then partitioned between aqueous hydrochloric acid (5M) and ethyl acetate. The organic layer was washed with brine, aqueous sodium bicarbonate, dried, and evaporated. The residue was chromatographed over silica gel (80g). Elution of the column with ethyl acetate-hexane (4:1) afforded 5-(E-2-bromovinyl)-3',5'-bis-O-isobutoxycarbonyl-2'-deoxyuridine (1.36g), m.p. 100-$101^{o}$ (from ether-hexane), λmax (EtOH) 249 (ε 14,500) and 292 (ε 12,400) nm; νmax (KBr) 1750, 1705, and 1250 $cm^{-1}$; $\delta_H$ ($CDCl_3$) 1.00 (12H, d, J 7Hz, $-CH_2CH(C\underline{H}_3)_2$), 1.83-2.75 (4H, m, 2'-CH, $-CH_2C\underline{H}Me_2$), 3.95 (2H, d, J 7Hz, $-C\underline{H}_2CHMe_2$), 4.05 (2H, d, J 7Hz, $-C\underline{H}_2CHMe_2$), 4.36 (1H, m, 4'-CH), 4.45 (2H, m, 5'-$CH_2$), 5.20 (1H, m, 3'-CH), 6.42 (1H, m, 1'-CH), 6.67 (1H, d, J 14Hz, C$\underline{H}$=CHBr), 7.45 (1H, d, J 14Hz, CH=C$\underline{H}$Br), 7.67 (1H, s, 6-CH), and 9.06 (1H, m, $D_2O$ exchangeable, NH) (Found: C, 47.55; H, 5.4; N, 5.2 %. $C_{21}H_{29}N_2O_9Br$ requires, C, 47.3; H, 5.5; N, 5.25 %), followed by 5-(E-2-bromovinyl)-5'-O-isobutoxycarbonyl-2'-deoxyuridine (1.37g), m.p. 162-$165^{o}$ (from hexane-ethyl acetate), λmax (EtOH) 249 (ε 12,600 and 293 (ε 11,000) nm; νmax (KBr) 1752, 1700, 1280, and 1240 $cm^{-1}$; $\delta_H$ [$(CD_3)_2SO$] 0.90 (6H, d, J 7Hz, $CH_2CH(C\underline{H}_3)_2$), 1.64-2.25 (3H, m, 2'-$CH_2$, $CH_2C\underline{H}Me_2$), 3.85 (2H, d, J 7Hz, $C\underline{H}_2CHMe_2$), 3.90 (1H, m, 4'-CH), 4.24 (3H, m, 5'-$CH_2$, 3'-CH), 5.40 (1H, m, $D_2O$ exchangeable, OH), 6.13 (1H, t, J 6Hz, 1'-CH), 7.81 (1H, d, J 14Hz, C$\underline{H}$=CHBr), 7.27 (1H, d, J 14Hz, CH=C$\underline{H}$Br), 7.75 (1H, s, 6-CH), and 11.55 (1H, m, $D_2O$ exchangeable, NH) (Found: C, 44.6; H, 4.65; N, 6.2 %. $C_{16}H_{21}N_2O_7Br$ requires C, 44.35; H, 4.9; N, 6.45 %).

## Examples 18, 19 and 20

**5-(E-2-Bromovinyl)-5'-O-t-butoxycarbonyl-2'-deoxyuridine (Example 18), 5-(E-2-Bromovinyl)-3',5'-bis-O-t-butoxycarbonyl-2'-deoxyuridine (Example 19) and 5-(E-2-Bromovinyl)-3'-O-t-butoxycarbonyl-2'-deoxyuridine (Example 20).**

p-Nitrophenyl t-butyl carbonate (3g) was added to a stirred mixture of 5-(E-2-bromovinyl)-2'-deoxyuridine (2.12g), triethylamine (2.2ml) and 4-dimethylaminopyridine (0.2g) in N,N-dimethylformamide at room temperature. The mixture was stirred at room temperature overnight. It was then partitioned between aqueous hydrochloric acid and ethyl acetate. The organic extract was washed with aqueous sodium carbonate, brine, dried, and evaporated. The residue was chromatographed over silica gel (80g). Elution of the column with ethyl acetate-hexane (3:1) gave two fractions. Fraction A contained a mixture of two compounds. Fraction B afforded 5-(E-2-bromovinyl)-5'-O-t-butoxycarbonyl-2'-deoxyuridine (0.6g); m.p. 170-172$^{o}$ (from ethyl acetate-hexane); λmax (EtOH) 250 (ε 14,800) and 293 (ε 12,400) nm; νmax (KBr) 1748, 1700, 1470, and 1290 $cm^{-1}$; $\delta_H$ [$(CD_3)_2SO$] 1.43 (9H, s, $(CH_3)_3C$), 2.16 (2H, t, J 6Hz, 2'-$CH_2$), 3.91 (1H, m, 4'-CH), 4.20 (3H, m, 3'-CH, 5'-$CH_2$), 5.41 (1H, m, $D_2O$ exchangeable, OH), 6.15 (1H, t, J 6Hz, 1'-CH), 6.84 (1H, d, J 14Hz, C<u>H</u>=CHBr), 7.31 (1H, d, J 14Hz, CH=C<u>H</u>Br), 7.77 (1H, s, 6-CH), and 11.60 (1H, m, $D_2O$ exchangeable, NH) (Found: C, 44.45; H, 4.7; N, 6.4 %. $C_{16}H_{21}N_2O_7Br$ requires C, 44.35; H, 4.9; N, 6.45 %).

Fraction A from above was re-chromatographed over silica gel (50g). Elution of the column with hexane-ethyl acetate (3:2) gave 5-(E-2-bromovinyl)-3',5'-bis-O-t-butoxycarbonyl-2'-deoxyuridine (0.4g); λmax (EtOH) 249 (ε 14,200) and 291 (ε 12,100) nm; νmax ($CHCl_3$) 1744, 1710, 1270, and 1150 $cm^{-1}$; $\delta_H$ ($CDCl_3$) 150 (9H, s, $(CH_3)_3C$), 1.54 (9H, s, $(CH_3)_3C$), 2.08-2.65 (2H, m, 2'-$CH_2$), 4.30 (3H, m, 4'-CH, 5'-$CH_2$), 5.11 (1H, m, 3'-CH), 6.40 (1H, dd, J 6Hz, J 8Hz, 1'-CH), 6.67 (1H, d, J 14Hz, C<u>H</u>=CHBr), 7.42 (1H, d, J 14Hz, CH=C<u>H</u>Br), 7.72 (1H, s, 6-CH), and 8.93 (1H, m, $D_2O$ exchangeable, NH) (Found: C, 47.5; H, 5.2; N, 5.1 %. $C_{21}H_{29}N_2O_9Br$ requires C, 47.3; H, 5.5; N, 5.25 %), and 5-(E-2-bromovinyl)-3'-O-t-butoxycarbonyl-2'-deoxyuridine (0.23g); λmax (EtOH) 249 (ε 13,700) and 292 (ε 11,700) nm; νmax ($CHCl_3$) 1710, 1460, 1270, 1150, and 1090 $cm^{-1}$; $\delta_H$ ($CDCl_3$) 1.50 (9H, s, $(CH_3)_3C$), 2.45 (2H, m, 2'-$CH_2$), 2.57 (1H, m, $D_2O$ exchangeable, OH), 3.95 (2H, m, 5'-$CH_2$), 4.17 (1H, m, 4'-CH), 5.20 (1H, m, 3'-CH), 6.26 (1H, t, J 6Hz, 1'-CH), 6.64 (1H, d, J 14Hz, C<u>H</u>=CHBr), 7.33 (1H, d, J 14Hz, CH=C<u>H</u>Br), 7.86 (1H, s, 6-CH), and 9.15 (1H, m, $D_2O$ exchangeable, NH) (Found: C, 44.3; H, 4.75; N, 6.15 %. $C_{16}H_{21}N_2O_7Br$ requires C, 44.35; H, 4.9; N, 6.45 %).

0095294

Example 21

5'-O-Allyloxycarbonyl-5-(E-2-bromovinyl)-2'-deoxyuridine

Allyl chloroformate (0.44ml) was added to a stirred solution of 5-(E-2-bromovinyl)-2'-deoxyuridine (1.06g) in pyridine (25ml) at $0^{o}$ and the mixture was stirred at room temperature for 1 h. The same work-up and chromatography as in Example 14 gave the title compound (0.45g), m.p. 173-175$^{o}$ (from ethyl acetate-hexane), λmax (EtOH) 249 (ε 14,500) and 293 (ε 12,400) nm; νmax (KBr) 1750, 1710, 1695, 1675, 1285, and 1265 $cm^{-1}$; $\delta_H$ [$(CD_3)_2SO$] 2.20 (2H, t, J 6.5Hz, 2'-$CH_2$), 3.95 (1H, m, 4'-CH), 4.26 (3H, m, 5'-$CH_2$, 3'-CH), 4.59 (2H, m), 5.15-5.47 (2H, m), 5.40 (1H, m, $D_2O$ exchangeable, OH), 5.73-6.05 (1H, m), 6.15 (1H, t, J 6.5Hz, 1'-CH), 6.85 (1H, d, J 14Hz, C<u>H</u>=CHBr), 7.29 (1H, d, J 14Hz, CH=C<u>H</u>Br), 7.77 (1H, s, 6-CH), and 11.60 (1H, m, $D_2O$ exchangeable, NH) (Found: C, 43.65; H, 3.95; N, 6.65 %. $C_{15}H_{17}N_2O_7Br$ requires C, 43.2; H, 4.1; N, 6.7 %).

Example 22

5'-O-Benzyloxycarbonyl-5-(E-2-bromovinyl)-2'-deoxyuridine

Benzyl chloroformate (1.38ml) was added to a solution of 5-(E-2-bromovinyl)-2'-deoxyuridine (2.14g) in pyridine (40ml) at $0^{o}$ and the mixture was then stirred at room temperature for 4 h. It was then partitioned between aqueous hydrochloric acid-ethyl acetate. The organic extract was washed with aqueous sodium bicarbonate, dried, and evaporated. The residue was then chromatographed over silica gel (100g). Elution of the column with ethyl acetate-hexane (4:1) gave the title compound (1.0g), m.p. $175-177^{o}$ (from ethyl acetate-n-hexane); λmax (EtOH) 250 (ε 12,000) and 293 (ε 14,400) nm; νmax (KBr) 1758, 1695, and 1280 $cm^{-1}$; $\delta_H$ [$(CD_3)_2SO$] 2.19 (2H, t, J 6Hz, 2'-$CH_2$), 3.96 (1H, m, 4'-CH), 4.30 (3H, m, 3'-CH, 5'-$CH_2$), 5.17 (2H, s, $OCH_2Ph$), 6.18 (1H, t, J 6Hz, 1'-CH), 6.89 (1H, d, J 14Hz, CH=CHBr), 7.31 (1H, d, J 14Hz, CH=CHBr), 7.41 (5H, s, ArH), 7.82 (1H, s, 6-CH), and 11.60 (1H, m, $D_2O$ exchangeable, NH) (Found: C, 48.95; H, 4.15; N, 6.15 %. $C_{19}H_{19}N_2O_7Br$ requires C, 48.85; H, 4.1; N, 6.0 %).

0095294

Example 23

5-(E-2-Bromovinyl)-5'-O-phenoxycarbonyl-2'-deoxyuridine

Phenyl chloroformate (0.9ml) was added to a stirred solution of 5-(E-2-bromovinyl)-2'-deoxyuridine (2g) in pyridine (40ml) at 0° and the mixture was allowed to come to room temperature. It was then stirred at room temperature for 0.5h. The mixture was partitioned between aqueous hydrochloric acid (2M)-ethyl acetate. The organic extract was washed with aqueous sodium bicarbonate, dried and evaporated to give an oil. Chromatography over silica gel (100g) then afforded the title compound (1.8g); λmax (EtOH) 249 (ε 13,800), and 293 (ε 11,500) $nm^{-1}$; νmax (KBr) 1768, 1700, 1282, 1255, and 1212 $cm^{-1}$; $\delta_H[(CD_3SO_2)]$ 2.18 (2H, t, J 6Hz, 2'-$CH_2$), 4.00 (1H, m, 4'-CH), 4.20-4.50 (3H, m, 3'-CH, 5'-$CH_2$), 5.55 (1H, m, $D_2O$ exchangeable, 3'-OH), 6.16 (1H, t, J 6Hz, 1'-CH), 6.86 (1H, d, J 14Hz, C<u>H</u>=CHBr), 7.13-7.50 (6H, m, CH=C<u>H</u>Br, ArH), 7.82 (1H, s, 6-CH), and 11.60 (1H, m, $D_2O$ exchangeable, NH) (Found: C, 47.75; H, 3.4; N, 5.95 %. $C_{18}H_{17}N_2O_7Br$ requires C, 47.7; H, 3.8; N, 6.2 %).

Example 24

5-(E-2-Bromovinyl)-2'-deoxy-5'-O-(p-nitrophenoxycarbonyl)uridine

p-Nitrophenyl chloroformate (4g) in dichloromethane (10ml) was added to a stirred solution of 5-(E-2-bromovinyl)-2'-deoxyuridine (5.37g) in pyridine (60ml) at $0^{o}$ and the mixture was stirred at $0^{o}$ for 1 h. The same work-up and chromatography as in Example 14 gave the title compound (2.2g), m.p. 196-$200^{o}$ (from ethyl acetate), λmax (EtOH) 251 (ε 20,900), 259 (ε 20,100), and 282 (ε 16,500) nm; νmax (KBr) 1775, 1710, 1680, 1525, and 1220 $cm^{-1}$; $\delta_H$ [$(CD_3)_2SO$] 2.23 (2H, t, J 6.5Hz, 2'-$CH_2$), 4.05 (1H, m, 4'-CH), 4.36 (1H, m, 3'-CH), 4.48 (2H, m, 5'-$CH_2$), 5.50 (1H, m, $D_2O$ exchangeable, OH), 6.20 (1H, t, J 6.5Hz, 1'-CH), 6.86 (1H, d, J 14Hz, CH=CHBr), 7.28 (1H, d, J 14Hz, CH=CHBr), 7.85 (2H, d, J 9Hz, ArH), 7.83 (1H, s, 6-CH), 8.32 (2H, d, J 9Hz, ArH), and 11.45 (1H, m, $D_2O$ exchangeable, NH) (Found: C, 43.5; H, 3.15; N, 8.25 %. $C_{18}H_{16}N_3O_9Br$ requires C, 43.4; H, 3.25; N, 8.45 %).

Example 25

5-(E-2-Bromovinyl)-2'-deoxy-5'-O-isopropoxycarbonyluridine

A mixture of 5-(*E*-2-bromovinyl)-2'-deoxy-5'-O-(*p*-nitrophenoxycarbonyl)uridine(0.66g), 4-dimethylaminopyridine (0.5g), and isopropanol (4ml) in pyridine (20ml) was stirred at room temperature for 8 days. The same work-up and chromatography as in Example 14 gave the title compound (0.25g), m.p. 165-168$^{o}$ (from acetone-hexane), λmax (EtOH) 249 (ε 14,800) and 293 (ε 12,700) nm; νmax (KBr) 1750, 1720, 1695, 1470, 1285, and 1265 cm$^{-1}$; $\delta_H$ [$(CD_3)_2SO$] 1.25 (6H, d, J 7.5Hz), 2.20 (2H, t, J 6.5Hz, 2'-$CH_2$), 3.92 (1H, m, 4'-CH), 4.27 (3H, m, 5'-$CH_2$, 3'-CH), 4.77 (1H, m), 5.43 (1H, m, $D_2O$ exchangeable, OH), 6.16 (1H, t, J 6.5Hz, 1'-CH), 6.85 (1H, d, J 14Hz, C<u>H</u>=CHBr), 7.32 (1H, d, J 14Hz, CH=C<u>H</u>Br), 7.77 (1H, s, 6-CH), and 11.60 (1H, m, $D_2O$ exchangeable, NH).

Example 26

5-(E-2-Bromovinyl)-2'-deoxy-5'-O-(2-hydroxyethoxycarbonyl)-uridine

Ethylene glycol (0.8ml) was added to a stirred mixture of 5-(*E*-2-bromovinyl)-2'-deoxy-5'-O-(*p*-nitrophenoxycarbonyl)-uridine (0.36g) in pyridine (5ml) and the mixture was stirred at room temperature for 5 days. The same work-up and chromatography as in Example 14 gave the title compound (0.11g), m.p. 160-168$^{o}$ (from ethanol-ether), λmax (EtOH) 249 (ε 14,000) and 293 (ε 12,300) nm; νmax (KBr) 1750, 1705, 1680, 1470, and 1285 $cm^{-1}$; $\delta_H$ [$(CD_3)_2SO$] 2.20 (2H, t, J 6.5Hz, 2'-$CH_2$), 3.60 (2H, m), 3.80-4.40 (6H, m), 4.84 (1H, m, $D_2O$ exchangeable, OH), 5.45 (1H, m, $D_2O$ exchangeable, OH), 6.16 (1H, t, J 6.5Hz, 1'-CH), 6.88 (1H, d, J 14Hz, C<u>H</u>=CHBr), 7.30 (1H, d, J 6.5Hz, CH=C<u>H</u>Br), 7.80 (1H, s, 6-CH), and 11.60 (1H, m, $D_2O$ exchangeable, NH) (Found: C, 39.7; H, 3.85; N, 6.55 %. $C_{14}H_{17}N_2O_8Br$ requires C, 39.9; H, 4.05; N, 6.65 %).

Example 27

2'-Deoxy-5'-O-ethoxycarbonyl-5-vinyluridine

To a solution of 2'-deoxy-5-vinyluridine (0.77g, 3.0mmol) in dry pyridine (6ml) at 0° was added ethyl chloroformate (0.41ml, 4.2mmol) and the solution was stirred for 0.5 h at 0°. The solution was partitioned between ethyl acetate and dilute hydrochloric acid and the aqueous layer was again extracted with ethyl acetate. The combined organic extracts were washed with aqueous sodium bicarbonate and dried (magnesium sulphate). On concentration of the ethyl acetate solution crystallisation took place to afford 2'-deoxy-5'-O-ethoxycarbonyl-5-vinyluridine as a white solid (0.53g, 54%), m.p. 145-147°; $\delta_H$ [$(CD_3)_2SO$] 1.24 (3H, t, J 7Hz, $CH_3$), 2.22 (2H, t, J 6Hz, 2'-H), 3.9-4.4 (6H, m, 3'-H, 4'-H, 5'-H and C<u>H</u>$_2CH_3$), 5.16 (1H, dd, J 3Hz and 12Hz, CH=C<u>H</u>H (<u>E</u>)), 5.46 (1H, br, $D_2O$ exchangeable, OH), 6.00 (1H, dd, J 3Hz and 18Hz, CH=CH<u>H</u> (<u>Z</u>)), 6.1-6.95 (1H, m, 1'-H and C<u>H</u>=CHH), 7.75 (1H, s, 6-H), and 11.45 (1H, br, $D_2O$ exchangeable, 3-H) (Found: C, 51.55; H, 5.7 N, 8.4 %. $C_{14}H_{18}N_2O_7$ requires C, 51.55; H, 5.55; N, 8.6 %).

## Antiviral Activity

### Method

Vero (African Green Monkey Kidney) cells were grown to confluence in 24 well multidishes, each well being 1.6cm in diameter. The cells were infected with Herpes simplex type 1 virus (HFEM strain) and overlaid with 0.5ml of 0.9% agarose (w/v) in maintenance medium. Test compounds prepared in maintenance medium in concentrations ranging from 100 to 0.03µg/ml in half-log dilution steps, were added in 0.5ml volume. The virus infected cultures were then incubated at $37^{o}$ for 4 days before fixing in 4% formaldehyde solution and staining with carbolfuchsin. The dishes were then examined to find what concentration of test compound caused a 50% reduction in the number of virus plaques formed ($PDD_{50}$ value) and the minimum concentration of test compound which caused cytotoxicity (MTD).

Results

| Example No. | $PDD_{50}$ µg/ml | $PDD_{50}$ µM | MTD (µg/ml) |
|---|---|---|---|
| 1 | 5.3 | 11.8 | >100 |
| 2 | 0.74 | 1.9 | >100 |
| 3 | 1.5 | 3.8 | >100 |
| 4 | 1.1 | 2.3 | >100 |
| 5 | 0.28 | 0.7 | >100 |
| 6 | 0.43 | 1.1 | >100 |
| 7 | 5.0 | 12.3 | >100 |
| 8 | 1.7 | 3.0 | >100 |
| 9 | 1.5 | 3.0 | >100 |
| 10 | 3.3 | 6.7 | >100 |
| 11 | 6.3 | 12.0 | >100 |
| 12 | 12.0 | 23.0 | >100 |
| 13 | 3.8 | 7.5 | >100 |
| 14 | 0.39 | 0.9 | >100 |
| 15 | 0.25 | 0.6 | >100 |
| 16 | 0.44 | 0.8 | 100 |
| 17 | 0.22 | 0.5 | >100 |
| 18 | 0.28 | 0.7 | >100 |
| 19 | 2.0 | 3.8 | >100 |
| 20 | 0.18 | 0.4 | >100 |
| 21 | 0.72 | 1.7 | >100 |
| 22 | 0.05 | 0.1 | >100 |
| 23 | 0.18 | 0.4 | >100 |
| 24 | 0.60 | 1.2 | 100 |
| 25 | 0.88 | 2.1 | >100 |
| 26 | 0.76 | 1.8 | >100 |
| 27 | 1.5 | 4.6 | >100 |

CLAIMS

1. A compound of formula (1):

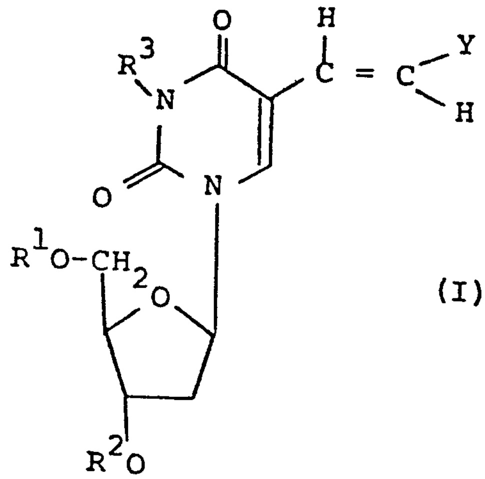

(I)

or a pharmaceutically acceptable salt or ester thereof, in which Y is a hydrogen or halogen atom, and each of $R^1$, $R^2$ and $R^3$ is a hydrogen atom; an acyl radical of the formula $X - \underset{\underset{O}{\|}}{C} -$ , in which X is a $C_{1-12}$ alkyl group, an optionally substituted phenyl, or optionally substituted benzyl group, or a carboxy group of the formula $HO - \underset{\underset{O}{\|}}{C} - Z -$ in which Z is a branched or straight chain alkylene radical having from 1 to 4 carbon atoms in the chain; an optionally substituted $C_{1-12}$ alkoxycarbonyl group; an optionally substituted $C_{3-12}$ alkenyloxycarbonyl group; an optionally substituted phenoxycarbonyl group; or an optionally substituted phenyl $C_{1-6}$ alkoxycarbonyl group; provided at least one of $R^1$, $R^2$ and $R^3$ is an optionally substituted alkoxycarbonyl, optionally substituted alkenyloxycarbonyl, optionally substituted phenoxycarbonyl or optionally substituted phenyl $C_{1-6}$ alkoxycarbonyl group.

2. A compound according to claim 1, in which $R^1$ $R^2$ or $R^3$ is a $C_{1-6}$ alkoxycarbonyl group.

3. A compound according to claim 2, in which $R^1$, $R^2$ or $R^3$ is methoxycarbonyl or ethoxycarbonyl.

4. A compound according to claim 1, in which $R^1$, $R^2$ or $R^3$ is an optionally substituted benzyloxycarbonyl group.

5. A compound according to any one of claims 1 to 4, in which X is $C_{1-6}$ alkyl or Z is $(-CH_2-)_n$ wherein n is 2 or 3.

6. A compound according to claim 1, selected from:

5-(E-2-Bromovinyl)-2'-deoxy-3',5'-bis-O-methoxycarbonyluridine;
5-(E-2-Bromovinyl)-2'-deoxy-3'-O-methoxycarbonyluridine;
5-(E-2-Bromovinyl)-2'-deoxy-5'-O-methoxycarbonyluridine;
5-(E-2-Bromovinyl)-2'-deoxy-3',5'-bis-O-ethoxycarbonyluridine;
5-(E-2-Bromovinyl)-2'-deoxy-5'-O-ethoxycarbonyluridine;
5-(E-2-Bromovinyl)-2'-deoxy-3'-O-ethoxycarbonyluridine;
5-(E-2-Bromovinyl)-2'-deoxy-3-ethoxycarbonyluridine;
5-(E-2-Bromovinyl)-2'-deoxy-5'-O-ethoxycarbonyl-3,3'-O-bisvaleryluridine;
5-(E-2-Bromovinyl)-2'-deoxy-5'-O-ethoxycarbonyl-3'-O-valeryluridine;
5-(E-2-Bromovinyl)-2'-deoxy-5'-O-ethoxycarbonyl-3-valeryluridine;
5-(E-2-Bromovinyl)-2'-deoxy-5'-O-ethoxycarbonyluridine 3'-Succinate Sodium Salt;
5-(E-2-Bromovinyl)-2'-deoxy-5'-O-ethoxycarbonyluridine

3'-Phosphate Disodium Salt;

5-(E-2-Bromovinyl)-2'-deoxy-5'-O-ethoxycarbonyluridine 3'-Phosphate Monosodium Salt;

5-(E-2-Bromovinyl)-2'-deoxy-5'-O-propoxycarbonyluridine;

5-(E-2-Bromovinyl)-5'-O-n-butoxycarbonyl-2'-deoxyuridine;

5-(E-2-Bromovinyl)-3',5'-bis-O-butoxycarbonyl-2'-deoxyuridine;

5-(E-2-Bromovinyl)-5'-O-isobutoxycarbonyl-2'-deoxyuridine;

5-(E-2-Bromovinyl)-5'-O-t-butoxycarbonyl-2'-deoxyuridine;

5-(E-2-Bromovinyl)-3',5'-bis-O-t-butoxycarbonyl-2'-deoxyuridine;

5-(E-2-Bromovinyl)-3'-O-t-butoxycarbonyl-2'-deoxyuridine;

5'-O-Allyloxycarbonyl-5-(E-2-bromovinyl)-2'-deoxyuridine;

5'-O-Benzyloxycarbonyl-5-(E-2-bromovinyl)-2'-deoxyuridine;

5-(E-2-Bromovinyl)-5'-O-phenoxycarbonyl-2'-deoxyuridine;

5-(E-2-Bromovinyl)-2'-deoxy-5'-O-(p-nitrophenoxycarbonyl)uridine;

5-(E-2-Bromovinyl)-2'-deoxy-5'-O-isopropoxycarbonyluridine;

5-(E-2-Bromovinyl)-2'-deoxy-5'-O-(2-hydroxyethoxycarbonyl)uridine;

2'-Deoxy-5'-O-ethoxycarbonyl-5-vinyluridine;

7. A process for preparing a compound of formula (I), which comprises reacting a compound of formula (IV)

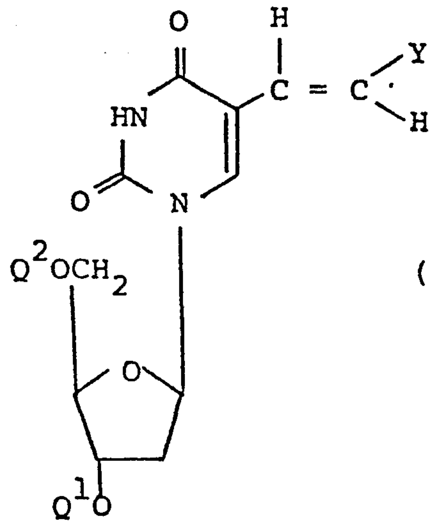

(IV)

wherein Y is as defined in formula (I) and $Q^1$ and $Q^2$ are each hydrogen or O-protecting groups, with an appropriate alkyl, alkenyl, phenyl or phenylalkyl-chloroformate, deprotecting the resultant product if necessary, and optionally thereafter treating the compound of formula (I) thus formed with an acylating agent containing the $X-\underset{\underset{O}{\|}}{C}-$ group or $-Z-\underset{\underset{O}{\|}}{C}-$ group, wherein X and Z are as defined in formula (I).

8. A process according to claim 7, in which $Q^1$ and $Q^2$ are both hydrogen, or one is hydrogen and the other is an O-protecting group.

9. A pharmaceutical composition which comprises a compound of the formula (I) or a pharmaceutically acceptable salt or ester thereof, together with a pharmaceutically acceptable carrier or excipient.

10. A compound according to claim 1 for use in the treatment of viral infections.

European Patent Office

# EUROPEAN SEARCH REPORT

0095294

Application number

EP 83 30 2740

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | DE-A-3 010 399 (K. GAURI) * Pages 5-7 * | 1-10 | C 07 H 19/08<br>A 61 K 31/70 |
| Y | DE-A-2 915 254 (UNIVERSITY OF BIRMINGHAM) * Pages 1-2 * | 1-10 | |
| P,D X | EP-A-0 061 283 (BEECHAM) * Pages 1-4; claims * | 1-10 | |
| E | EP-A-0 082 668 (BEECHAM) | 1-10 | |

TECHNICAL FIELDS SEARCHED (Int Cl 3)

C 07 H 19/00
A 61 K 31/00

The present search report has been drawn up for all claims

| Place of search | Date of completion of the search | Examiner |
|---|---|---|
| THE HAGUE | 26-08-1983 | VERHULST W. |

CATEGORY OF CITED DOCUMENTS

X particularly relevant if taken alone
Y particularly relevant if combined with another document of the same category
A technological background
O non-written disclosure
P intermediate document
T : theory or principle underlying the invention
E : earlier patent document, but published on, or after the filing date
D : document cited in the application
L : document cited for other reasons
& : member of the same patent family, corresponding document

EPO Form 1503 03 82